# EUROPEAN PATENT APPLICATION

(11) **EP 4 588 444 A1**
(43) Date of publication of application: **23.07.2025**
(21) Application number: 22957620.2
(22) Date of filing: 05.09.2022
(51) Int. Cl.: A61B 17/04, A61B 17/06, A61B 17/062

(54) **CAPSULE NEEDLE AND THREAD MODULE**

(71) Applicant: MATRIXLabs Medical Inc., New Taipei City, 244019 (TW)
(72) Inventor: TU, Fung-Chao, New Taipei City, Taiwan 22060 (TW); WU, Wen-Yih, Taipei, Taiwan 10550 (TW); SUNG, Ching Chun, New Taipei City, Taiwan 220 (TW); WANG, Chung Chih, New Taipei City, Taiwan 220 (TW); SU, Jimmy Chunmin, Kaohsiung City, Taiwan 81356 (TW); KUO, Chia-Sheng, NewTaipei City, Taiwan 244 (TW); POLAK, Pim David, 3743 Baarn (NL)
(74) Representative: Cabinet Chaillot
(86) International application number: PCT/CN2022/117103
(87) International publication number: WO 2024/050670

(57) **Abstract**

A capsule knotting module has an operating member (10) and a knotting member (20). With a suture (A), a pre-knot (A1) and a sliding loop (A2) are formed at a front end of the knotting member (20). When in use, the pre-knot (A1) is tied to form a secure knot at a front end of the knotting member (20) to achieve the purpose of suturing and fixing a tissue wound.

## Description

### FIELD OF INVENTION

The present invention relates to a knotting assembly, and more particularly to a capsule knotting module.

### BACKGROUND OF THE INVENTION

With the advancement of modern medical technology, surgical techniques have also evolved toward minimally invasive approaches, such as small incisions or minimally invasive procedures. These not only facilitate rapid and effective wound healing but also help reduce and improve post-operative scarring. In a conventional knot-tying process, when a surgeon aims to close a wound, they typically use a handheld surgical needle with an attached suture. The tissue on both sides of the wound is sutured and tightened, forming a secure knot to anchor the end of the suture.

However, it is complex and difficult to perform the conventional knot-tying process manually by to wrap the surgical suture and to form the secure knot during a minimally invasive surgery, which usually results in a poor quality of the secure knot. In addition, it is inefficient to tie multiple secure knots by the conventional method and it increases the risk of wound dehiscence while the secure knots are under poor quality.

### SUMMARY OF THE INVENTION

To overcome the issues associated with the conventional knotting techniques used in surgical wound closures, such as the inefficiency of repeated operations to form multiple secure knots and the risk of wound dehiscence affecting the patient's recovery efficiency.

The present invention provides a capsule knotting module comprising a knotting member having a through hole formed through the knotting member. Two ends of the through hole are defined respectively as a front end and a rear end. A shaft extends through the through hole and protrudes from the front end of the through hole. A suture extends through the through hole and the shaft and has two ends defined respectively as an operating end and a knotting end. The operating end and the knotting end are respectively exposed out of the rear end of the through hole and a front end of the shaft.

Wherein, at least a part of the knotting end of the suture is wrapped around an outer circumference of the shaft and forms a pre-knot, and a sliding loop is formed between the shaft and the pre-knot. A size of the sliding loop is varied according to a distance between the operating member and the knotting member.

Wherein, a diameter of a knotted structure is larger than a diameter of the through hole, and the knotted structure is stopped at the rear end of the through hole.

Wherein, the knotted structure comprises a spherical knot or comprises a spherical knot with a pulling ring connected to a rear end of the spherical knot.

Wherein, the knotting member comprises a large-diameter section formed at the rear end of the through hole, a diameter of the large-diameter section is smaller than or equal to a diameter of the knotted structure to allow at least a part of the knotted structure to be detachably held in the large-diameter section.

Wherein, a tying portion is defined on at least a part of the shaft protruding from the through hole, and a length of the tying portion is varied according to a relative position of the shaft and the through hole. During an operation, at least a part of the suture extends through the sliding loop, and the sliding loop is pulled to be shrunk and abuts with the outer circumference of the shaft. The sliding loop continues to be shrunk and pushes the shaft to move into the through hole to decrease the length of the tying portion. When the shaft is completely moved inside the through hole, and the pre-knot is detached from the shaft and is tied with the sliding loop and at least a part of the suture to form a secure knot.

Wherein, at least a part of the shaft is securely held in the through hole , wherein:
during an operation, at least a part of the suture extends through the sliding loop, and the sliding loop is pulled to be shrunk and abuts with the outer circumference of the shaft;
the operating member is continuously pulled to make the pre-knot to move toward the front end of the capsule knotting module until the pre-knot is detached from the shaft ; and
the pre-knot is detached from the shaft and is tied with the sliding loop and at least a part of the suture, and a secure knot is formed.

Wherein, the knotting end of the suture is connected with a surgical needle.

When the capsule knotting module is in use, a user aligns a front end of the knotting member with an intended suture site and the surgical needle is passed through the intended suture site. The surgical needle with the suture extends through the sliding loop. Once suturing is complete, the operating member is moved away from the knotting member to allow the suture to be pulled by the operating member and to make the sliding loop be shrunk until it adheres to the outer circumference of the shaft.

Continuously pulling the operating member and/or simultaneously pulling the surgical needle, a tension is generated at two ends of the suture to make the pre-knot be escape from the shaft. Thus, the sliding loop is tied with the suture extending through the sliding loop to form a secure knot.

Compared with the current technology, the advantage of the present invention comprises:
The capsule knotting module of the present invention has a simple structure, and can be designed in various suture size as needed. It can be miniaturized for fit a minimally invasive surgery, and assisting the operators in completing suturing and knotting procedures seamlessly in narrow spaces, optimizing the surgical time and the quality of knotting. Furthermore, by extending its length while minimizing suture usage, the capsule knotting module reduces costs effectively.

The stop portion assists operators in aligning the capsule knotting module with the intended suture site while preventing the shaft from directly contacting the suture and avoiding contamination by blood or tissue fluid. Additionally, the stop portion effectively prevent the pre-knot from slipping from the shaft unexpectedly

Particularly when the shaft is disposed in the hole, during the formation of the secure knot, the operator must apply sufficient tension by pulling the operating member and /or the surgical needle. This tension causes the pre-knot to push against and deform the stop portion, allowing it to slip from the shaft. During this process, the stop portion can prevent the pre-knot from slipping off from the sleeve shaft in advance, thereby preventing an undesirable knot location or poor knot quality that could result in looseness or failure.

### IN THE DRAWINGS

Fig. 1 is a perspective view of a first preferred embodiment of a capsule knotting module in accordance with the present invention;
Fig. 2 is an exploded perspective view of the capsule knotting module in Fig. 1;
Fig. 3a is a first operating side view in partial section of the capsule knotting module in Fig. 1;
Fig. 3b is a second operating side view in partial section of the capsule knotting module in Fig. 1;
Fig. 4 is a perspective view in partial section of a second preferred embodiment of a capsule knotting module in accordance with the present invention;
Fig. 5 is a perspective view of a third preferred embodiment of a capsule knotting module in accordance with the present invention;
Fig. 6 is an exploded perspective view of the capsule knotting module in Fig. 5;
Fig. 7a is a first operating side view in partial section of the capsule knotting module in Fig. 5;
Fig. 7b is a second operating side view in partial section of the capsule knotting module in Fig. 5;
Fig. 8a is a perspective view of a fourth preferred embodiment of a capsule knotting module in accordance with the present invention; and
Fig. 8b is a side view in partial section of the capsule knotting module accordance with the present invention.

### Symbol description:

10 operating member
11 connecting rib
111 raised part
12 pull rod
13 operating portion
131 extended arm
20 knotting member
21 through hole
211 large-diameter section
22 shaft
221 tying portion
23 connecting portion
231 cavity
232 groove
235 notch
24 limiting hole
25 stop portion
251 extension section
252 stopping section
26 holding space
30 housing
A suture
A1 pre-knot
A2 sliding loop
A3 knotted structure
A31 spherical knot
A32 pulling ring
B surgical needle
C limiting suture
C1 limiting loop

### DETAILED DESCRIPTION OF THE PREFERRED EMBODIMENTS

In order to make purposes, technical solutions, and advantages of the present invention to be clearer, the following content provides some preferred embodiments in accordance with the present invention in cooperation with the drawings to clearly and completely describe the techniques of the present invention. The following embodiments are only a part of embodiments of the present invention, but not all the embodiments of the present invention. Based on the following embodiments of the present invention, the person having an ordinary skill in the art can get embodiments without any inventive work that are all the embodiments of the present invention.

The present invention further described in detail below in conjunction with the drawings:

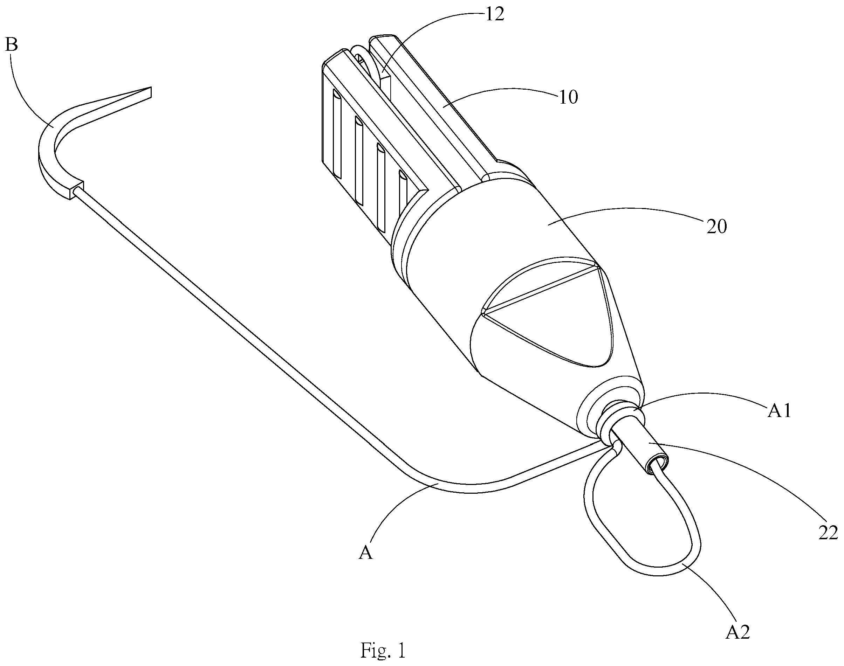

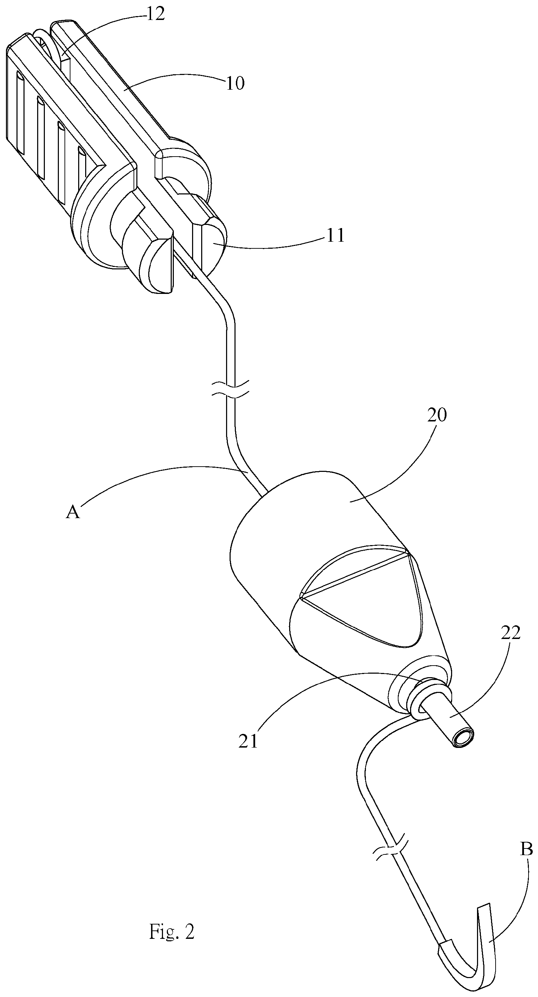

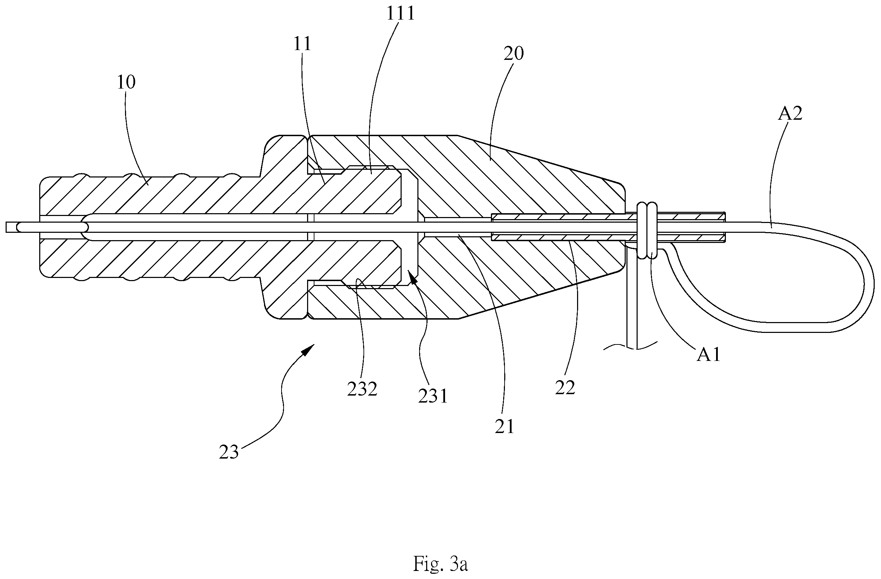

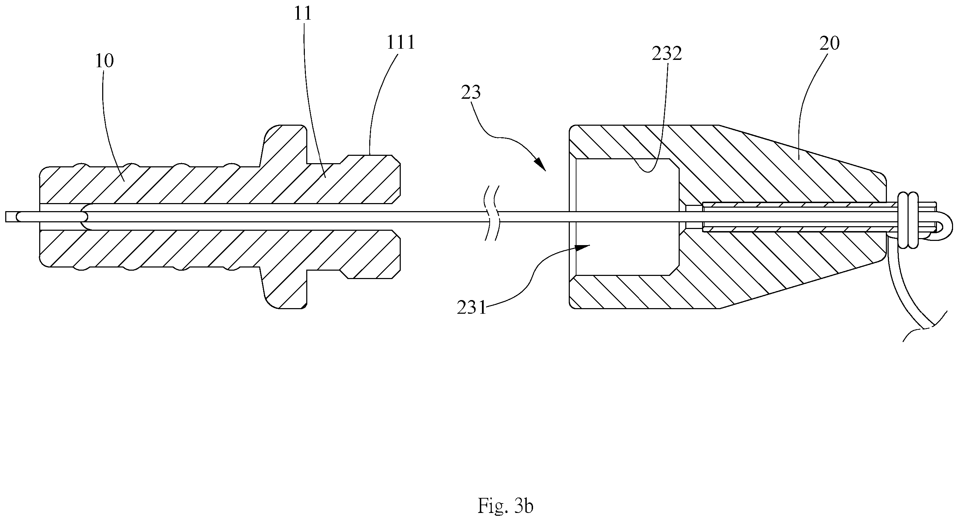

Refer to Figs. 1 to 3b, a first preferred embodiment of a capsule knotting module in accordance with the present invention is illustrated and comprises an operating member 10 and a knotting member 20. The operating member 10 is formed on a rear end of the capsule knotting module, and the knotting member 20 is formed on a front end of the capsule knotting module. The knotting member 20 comprises a through hole 21 defined through the knotting member 20, and a suture A extends through the through hole 21. An operating end of the suture A is connected to a part of the operating member 10. Means for connecting the operating end of the suture A and a part of the operating member 10 is not limited in the present invention. Methods, such as gluing or binding are all included in the scope of the present invention. In the embodiment, the operating member 10 comprises a pull rod 12 arranged diametrically, and the operating end of the suture A is bound on the pull rod 12.

A shaft 22 is a hollow tubular, is disposed in the through hole 21, and protrudes from the front end of the through hole 21 of the knotting member 20. An end opposite the operating end of the suture A is defined as a knotting end. After the knotting end of the suture A extends through the through hole 21 and the shaft 22, the knotting end extends toward and is exposed from the front end of the capsule knotting module.

The knotting end of the suture A exposed of the capsule knotting module is wrapped around an outer circumference of the shaft 22 to form a pre-knot A1 and a sliding loop A2 formed between the shaft 22 and the pre-knot A1. A surgical needle B is connected to an end of the knotting end of the suture A for suturing and fixing a tissue wound.

In the first embodiment, at least a part of the shaft 22 is securely disposed in the through hole 21. When the capsule knotting module is in use, a user aligns a front end of the knotting member 20 with an intended suture site and the surgical needle B is passed through the intended suture site, and the surgical needle B with the suture A/the knotting end extends through the sliding loop A2. Furthermore, during suturing process, a second suture different from the suture A can be applied to suture the intended suture site and extends through the sliding loop A2. After suturing process, the user moves the operating member 10 away from the knotting member 20, the operating end of the suture A is pulled, thereby the sliding loop A2 is shrunk until at least a part of the sliding loop A2 abutting against the outer circumference of the shaft 22 and tighten around the shaft 22. Then the operating member 10 is continuously pulled and/or the surgical needle B is simultaneously pulled, tensions generated on two ends of the suture A to make the pre-knot A1 move forward and escape from the shaft 22. Thus, the sliding loop A2 and the suture A extending through the sliding loop A2 are tied together to form a secure knot.

In another preferred embodiment, the shaft 22 can be slid along the through hole 21, and a length of the shaft 22 protruding from a front end of the through hole 21 can be adjusted according to a position of the shaft 22 relative to the through hole 21. The at least a part of the shaft 22 protruding from the through hole 21 is defined as a tying portion 221. When the operating member 10 is kept being pulled away from the knotting member 20, the sliding loop A2 is constricted and urge the shaft 22 to move inside the through hole 21. Thus, the length of the tying portion 221 is gradually shorten. When the tying portion 221 is completely moved into the through hole 21, the pre-knot A1 is slipped off the shaft 22 and the sliding loop A2 and the suture A extending through the sliding loop A2 are tie together to form the secure knot.

In another embodiment, the knotting member 20 comprises a connecting portion 23 for being detachably assembled with the operating member 10 to prevent the operating member 10 from being pulled unexpectedly. The way for assembling the operating member 10 with the connecting portion 23 is not limited in the present invention, methods such as concave-convex matching or providing a connector detachably arranged between the connecting portion 23 and the operating member 10 are all included in the scope of the present invention.

In this embodiment, a cavity 231 is defined in a rear end of the knotting member 20 and is served as the connecting portion 23, and a groove 232 is annually defined in at least a part of an inner wall of the cavity 231. The operating member 10 comprises two connecting ribs 11 protruding from the front end of the operating member 10 and spaced with each other. A farthest distance between two connecting ribs 11 is smaller than a diameter of the cavity 231. The two connecting ribs 11 on the operating member 10 can be inserted detachably into the cavity 231 in the knotting member 20. Preferably, the cavity 231 in the knotting member 20 communicates with the rear end of the through hole 21. The two connecting ribs 11 protrude from the front end of the operating member 10 from the two ends of the pull rod 12. The operating end of the suture A extends through the through hole 21, the cavity 231, and a gap between the two connecting ribs 11 in sequence and is tied with the pull rod 12.

A raised part 111 diametrically protrudes from a front end of each connecting rib 11 at a position corresponding to the groove 232. When the two connecting ribs 11 are inserted into the cavity 231, the raised parts 111 are held in the groove 232 to keep the operating member 10 from being separated from the knotting member 20 with an engagement between the raised parts 111 and the groove 232.

During the operation, to disengage the operating member 10 from the knotting member 20, a force is applied to the two connecting ribs 11 to move the connecting ribs 11 toward each other. Thus, the engagement between the raised parts 111 and the groove 232 can be released, such that the operating member 10 can be disengaged from the knotting member 20. The capsule knotting module of the present invention has a simple structure and can be designed in various suture size as needed. It can be miniaturized for fit a minimally invasive surgery, and assisting operators in completing suturing and knotting procedures seamlessly in narrow spaces, optimizing the surgical time and the quality of knotting.

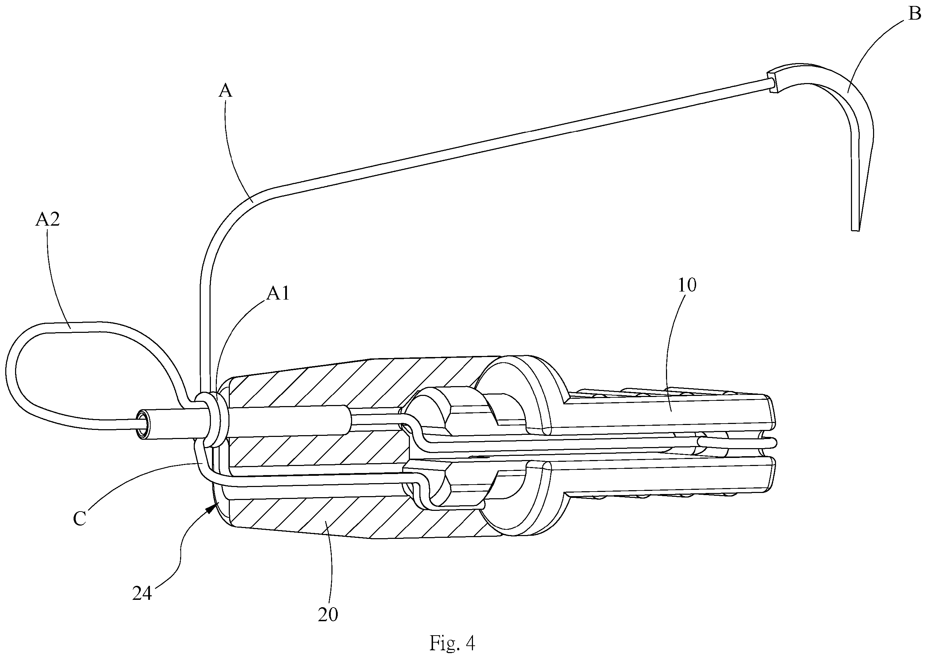

With reference to Fig. 4, a second preferred embodiment is illustrated. A difference between the second embodiment from the first embodiment is that the front end of the knotting member 20 further includes a limiting hole 24, a limiting suture C extends toward the front end of the knotting member 20 and out from the limiting hole 24. A limiting loop C1 is formed on at least a part of the limiting suture C protruding out of the limiting hole 24. At least a part of the limiting loop C1 is hooked with the pre-knot A1 and/or the sliding loop A2 to prevent the pre-knot A1 and the sliding loop A2 from being separated from the shaft 22 under unexpected conditions.

In this embodiment, the through hole 21 and the limiting hole 24 are parallel to each other and formed through the knotting member 20. The rear ends of the through hole 21 and the limiting hole 24 communicate with the cavity 231. The limiting suture C is folded and held in the limiting hole 24, and two ends of the limiting suture C extend toward the rear end of the limiting hole 24 and into the cavity 231. The limiting loop C1 formed by being folded extends toward the front end of the limiting hole 24 and is hooked with the pre-knot A1. When the operating member 10 is assembled with the connecting portion 23 of the knotting member 20 in a concave-convex manner, at least a part of the operating member 10 compress the two ends of the limiting suture C between the connecting portions 23. Thus, the limiting suture C only can be released after the operating member 10 is separated from the knotting member 20, and the pre-knot A1 is released at the same time and the secure knot is formed subsequently.

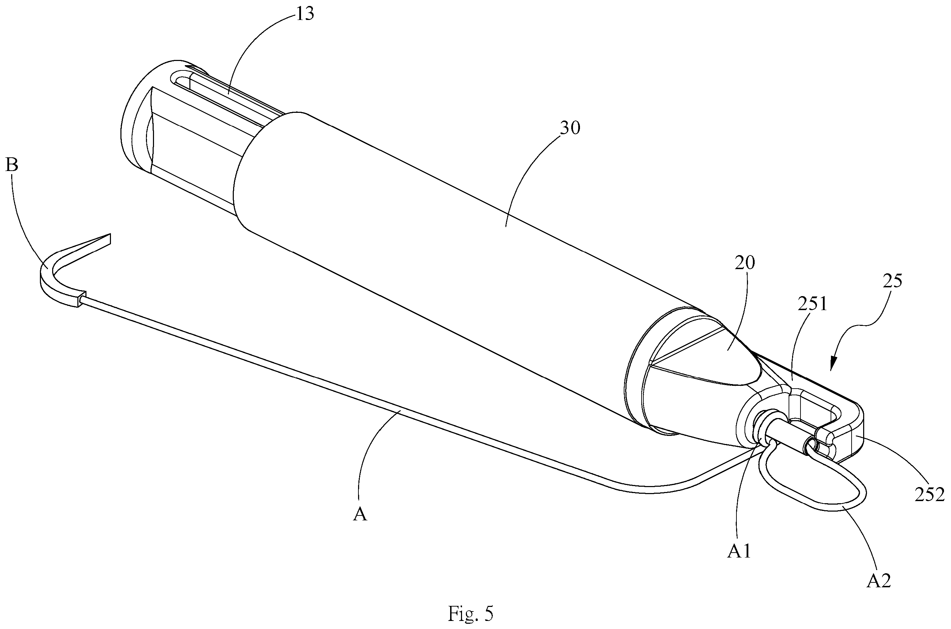

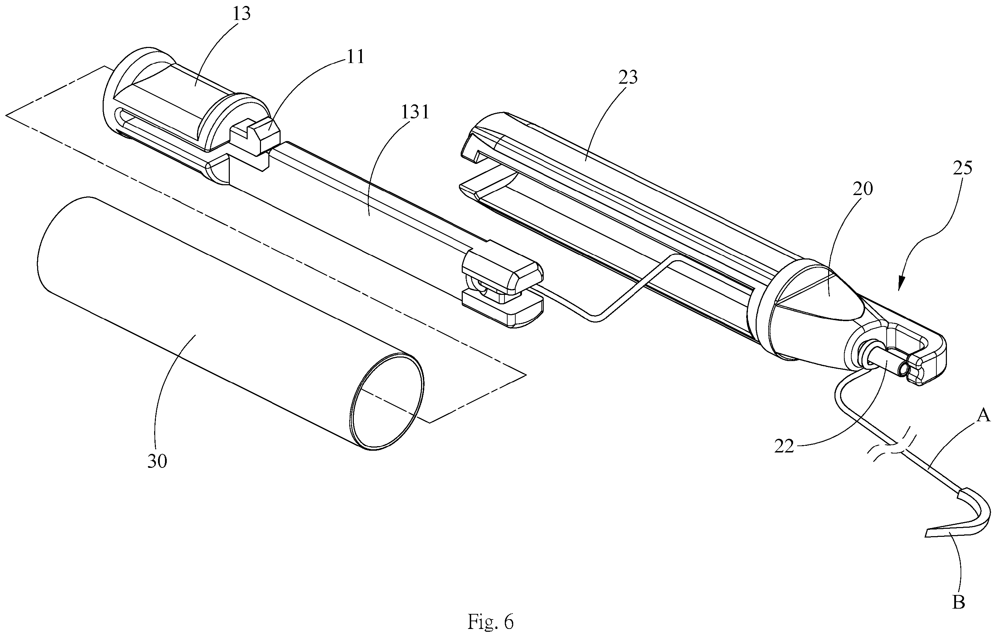

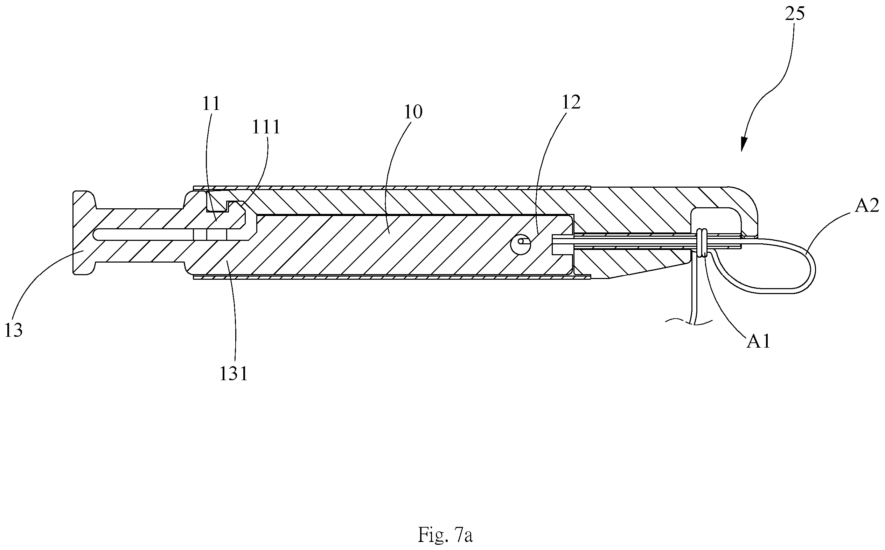

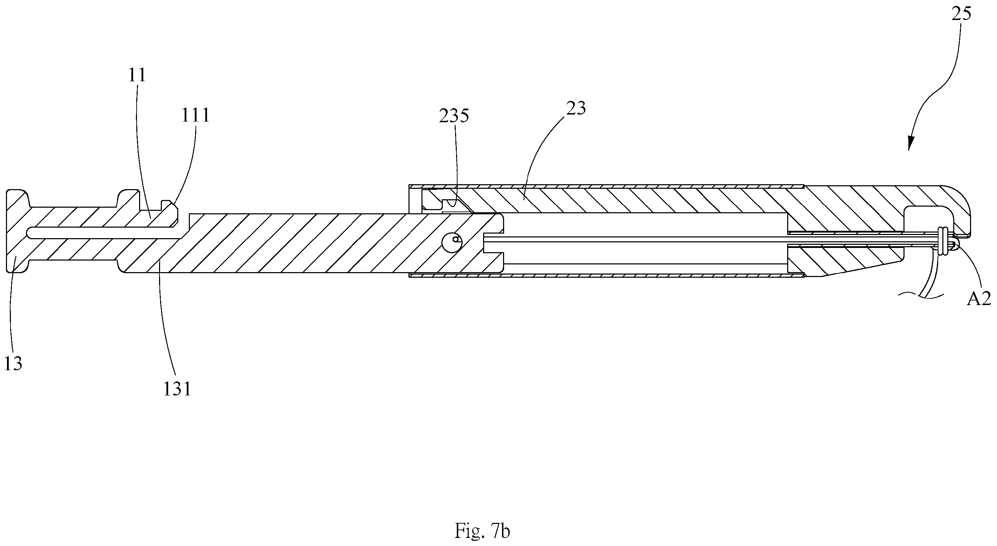

Further referring to a third preferred embodiment provided in Figs. 5 to 7b, the capsule knotting module comprises the operating member 10, the knotting member 20, and a housing 30. The housing 30 covers at least a part of the front end of the operating member 10 and at least a part of the rear end of the knotting member 20. The difference from the aforementioned embodiments is that the capsule knotting module is rod-shaped, this can minimize the use of suture A while increasing the length of the capsule knotting module. The capsule knotting module can be designed into different sizes according to requirements and optimize cost-effectiveness.

The operating member 10 is rod-shaped. The pull rod 12 is formed on the front end of the operating member 10 and is connected with the operating end of the suture A. The front end of the operating member 10 may slide relative to the knotting member 20 in the housing 30, and the rear end of the operating member 10 protrudes out of the housing 30 to form an operating portion 13 applied for enabling the user to hold the operating portion 13 to pull the operating member 10 to slide relative to the knotting member 20.

The capsule knotting module further comprises the connecting portion 23. The position of the connecting portion 23 is not limited, may be located between the operating member 10 and the knotting member 20, or between the operating member 10 and the housing 30. Structures or components for preventing the operating member 10 from being separated from the knotting member 20 unexpectedly are all included in the scope of the present invention.

Preferably, the connecting portion 23 is formed on the rear end of the knotting member 20 and extends into and is held in the housing 30. At least a part of the connecting portion 23 and at least a part of the front end of the operating member 10 are overlapped with each other in the housing 30. The connecting portion 23 may be the cavity 231 or a track, such that the front end of the operating member 10 is slidable in the housing 30 relative to the connecting portion 23.

In this embodiment, the connecting portion 23 is extends toward the rear end to a position adjacent to the operating portion 13. A notch 235 is defined in a surface of the connecting portion 23 corresponding to the operating member 10. An extending arm 131 and the connecting rib 11 protrude from the front end of the operating portion 13. The extending arm 131 and connecting rib 11 are spaced from each other. The connecting rib 11 is arranged at a position corresponding to the connecting portion 23.

Wherein, a front end of the extending arm 131 extends into the housing 30 and is connected with the pull rod 12. The front end of the connecting rib 11 extends into the housing 30 and the raised part 111 is raised toward to the notch 235. Accordingly, the operating member 10 is kept from be disengaged from the knotting member 20 with the engagement between the raised part 111 and the notch 235. To disengage the operating member 10 from the knotting member 20, a pressure is applied to the connecting rib 11 to deform the connecting rib 11 and to move the connecting rib 11 toward the extending arm 131, such that the raised part 111 can be escaped from the cavity 231 to release the concave-convex engagement relationship.

In another embodiment, an outer diameter of the knotting member 20 is tapered from the rear end to the front end so as to help the user to confirm a current position of the front end of the knotting member 20. Thus, the capsule knotting module can be prevented from being interfered with the user's field of vision when aligning the knotting member 20 with the intended suture site.

In another embodiment, a stop portion 25 is formed on the front end of the knotting member 20, and a holding space 26 is defined between the front end of the knotting member 20 and the stop portion 25. Preferably, the stop portion 25 comprises an extension section 251 extending from the front end of the knotting member 20 at a surface of the front end of the knotting member 20, and a stopping section 252 protrudes diametrically from the front end of the extension section 251 and corresponds to the through hole 21 in position. The holding space 26 is defined between the through hole 21, the extension section 251, and the stopping section 252, and a size of the holding space 26 can be defined by the length of the extension section 251.

When not in use, the length of the tying portion 221 of the shaft 22 is equal to the length of holding space 26. Preferably, at least a part of the outer circumference of the rear end of the tying portion 221 can be supported by the stopping section 252 to enhance the structural strength of the capsule knotting module. Furthermore, the outer circumference of the rear end of the tying portion 221 abuts with an end surface of the stopping section 252 to help the user to align the capsule knotting module with the intended suture site. Accordingly, the shaft 22 can be kept from being in directly contact with the suture and being contaminated with blood or tissue fluid. At the same time, while the stop portion 25 is supported by the shaft 22, the pre-knot A1 can be effectively prevented from being slipped from the shaft 22 unexpectedly.

It should be noted. in the embodiment of that the shaft 22 is securely disposed in the through hole 21 and during the process of forming the secure knot, sufficient tension is required for pulling the operating member 10 and/or the surgical needle B. Thus, the pre-knot A1 can push the stop portion 25 to be deformed to allow the pre-knot A1 to slip away from the shaft 22. During this process, the stop portion 25 can prevent the pre-knot A1 from being slipped off from the shaft 22 in advance, and the secure knot is prevented from being in an undesirable position or being of poor quality and easy to be loosened.

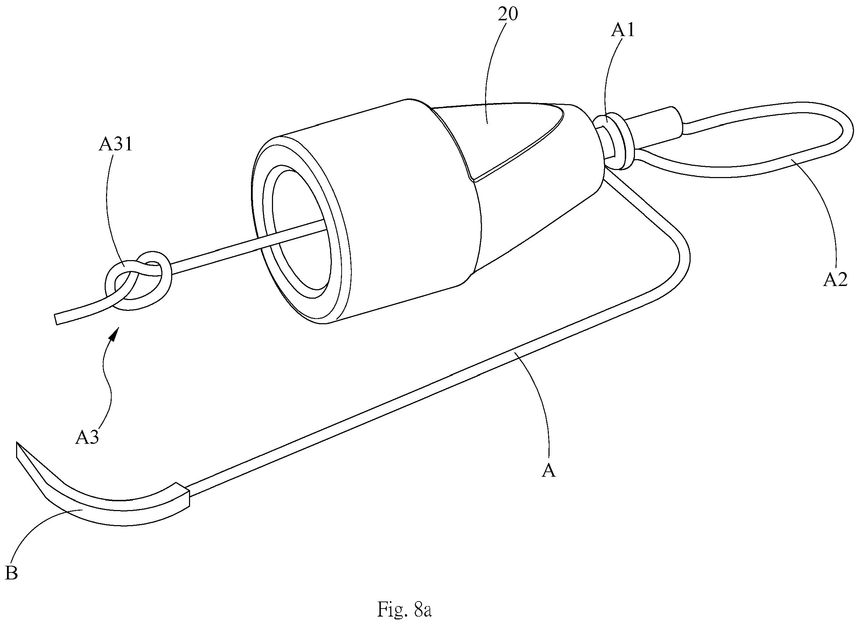

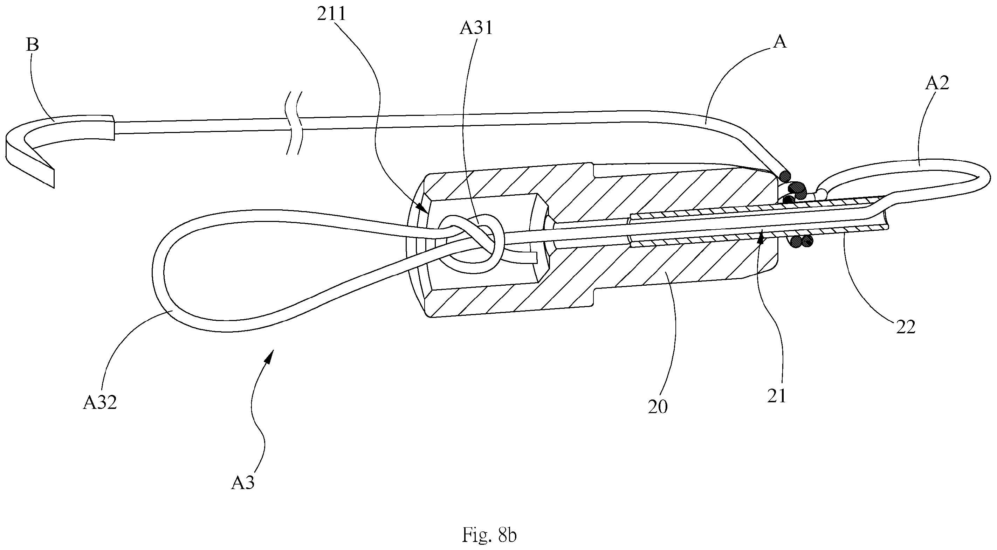

Further referring to the Fig. 8a and Fig. 8b, which are respectively the fourth and fifth preferred embodiments in accordance with the present invention. The difference from the above-mentioned embodiments is that the operating member 10 is formed by the operating end of the suture A. In the embodiments shown in Figs. 8a and 8b, the capsule knotting module comprises the knotting member 20 having the through hole 21 formed through the knotting member 20. Two ends of the through hole 21 are respectively defined as the front end and the rear end. The shaft 22 protrudes from the front end of the capsule knotting module. The structural relationship between the shaft 22 and the through hole 21 has been mentioned in above paragraphs and will not be omitted.

The suture A extends through the through hole 21 and the shaft 22, and two ends of the suture A are defined respectively as the operating end and the knotting end. Wherein, the operating end of the suture A is exposed from the rear end of the through hole 21, and the knotting end of the suture A is exposed from the front end of the shaft 22. Similarly, at least a part of the knotting end of the suture A is wrapped around the outer circumference of the shaft 22 to form the pre-knot A1, and the sliding loop A2 is formed between the shaft 22 and the pre-knot A1.

A knotted structure A3 is formed on at least a part of the operating end of the suture A, which is located at the rear end of the knotting member 20 and is served as the operating member 10. Accordingly, the size of the sliding loop A2 is varied according to a distance between the knotted structure A3 and the knotting member 20. Thus, the operating member 10 as described in the above-mentioned embodiments can be omitted to allow the present invention to be applied to minimally invasive surgeries such as laparoscopy or da Vinci Surgical System.

As shown in Fig. 8a, the knotted structure A3 is a spherical knot A31 formed at the operating end of the suture A. Preferably, a diameter of the spherical knot A31 is larger than the diameter of the through hole 21, so that the spherical knot A31 is kept from passing through the through hole 21 and the knotting member 20 abuts with the rear end of the through hole 21. When in use, the spherical knot A31 can be directly pulled, and the sliding loop A2 can be shrunk to form the secure knot.

The difference of the embodiment shown in Fig. 8b from the embodiment shown in Fig. 8a is that the knotted structure A3 comprises the spherical knot A31 and a pulling ring A32. During forming the knotted structure A3 in this embodiment, the operating end of the suture A can be folded at any position on the operating end, such that the rear end of the operating end is overlapped with at least a part of the operating end of the suture A to form the spherical knot A31. At the time of forming the spherical knot A31, the folded portion of the operating end of the suture A may be further defined as the pulling ring A32. When in use, the knotted structure A3 can be pulled by a tool hooked with the pulling ring A32, such that the convenience and accuracy of using the capsule knotting module can be improved and the knotted structure A3 can be kept from being slipped off while being pulled.

The knotted structure A3 can also be presented in different ways according to different demands. For example, a rod or an elastic ring can be tied with the spherical knot A31. Accordingly, the capsule knotting module can easily enter a body through the minimally invasive incision, and can also conveniently pull the knotted structure A3 and form the secure knot.

To avoid the knotted structure A3 being pulled unintentionally and to prevent the sliding loop A2 from being shrunk to cause the surgical needle B and the suture A to extend through the sliding loop A2 difficultly, a large-diameter section 211 is formed in the knotting member 20 at the rear end of the through hole 21. The knotting structure A3 is held in the large-diameter section 211.

Preferably, a diameter of the large-diameter section 211 is smaller than or equal to that of the spherical knot A31, and the spherical knot A31 on the knotted structure A3 is held in the large-diameter section 211. When in storage, a friction is formed between the spherical knot A31 and at least a part of a side wall of the large-diameter section 211, and the pulling ring A32 is exposed from the rear end of the large-diameter section 211. Thus, the knotted structure A3 can be held in and be kept from falling out easily from the large-diameter section 211. The knotted structure A3 can be kept from being moved relative to the knotting member 20 even if the knotting structure A3 and/or the pulling ring A32 is unintentionally touched, such that the sliding loop A2 can be prevented from being unintentionally shrunk.

Even though numerous characteristics and advantages of the present invention have been set forth in the foregoing description, together with details of the structure and function of the invention, the disclosure is illustrative only, and changes may be made in detail, especially in matters of shape, size, and arrangement of parts within the principles of the invention to the full extent indicated by the broad general meaning of the terms in which the appended claims are expressed.

## Claims

1. A capsule knotting module, **characterized in that** the capsule knotting module comprises:
a knotting member (20) having a through hole (21) formed through the knotting member (20), two ends of the through hole (21) defined respectively as a front end and a rear end, and a shaft (22) extending through the through hole (21) and protruding from the front end of the through hole (21);
a suture (A) extending through the through hole (21) and the shaft (22) and having two ends defined respectively as an operating end and a knotting end, and the operating end and the knotting end respectively exposed out of the rear end of the through hole (21) and a front end of the shaft (22), wherein
at least a part of the knotting end of the suture (A) is wrapped around an outer circumference of the shaft (22) and forms a pre-knot (A1), and
a sliding loop (A2) is formed between the shaft (22) and the pre-knot (A1); and
a size of the sliding loop (A2) is varied according to a distance between an operating member (10) and the knotting member (20).

2. The capsule knotting module as claimed in claim 1, wherein a diameter of a knotted structure (A3) is larger than a diameter of the through hole (21), and the knotted structure (A3) is stopped at the rear end of the through hole (21).

3. The capsule knotting module as claimed in claim 2, wherein the knotted structure (A3) comprises a spherical knot (A31) or comprises a spherical knot (A31) with a pulling ring (A32) connected to a rear end of the spherical knot (A31).

4. The capsule knotting module as claimed in any one of claims 1 to 3, wherein the knotting member (20) comprises a large-diameter section (211) formed at the rear end of the through hole (21), a diameter of the large-diameter section (211) is smaller than or equal to a diameter of the knotted structure (A3) to allow at least a part of the knotted structure (A3) to be detachably held in the large-diameter section (211).

5. The capsule knotting module as claimed in any one of claims 1 to 3, wherein a tying portion (221) is defined on at least a part of the shaft (22) protruding from the through hole (21), and a length of the tying portion (221) is varied according to a relative position of the shaft (22) and the through hole (21), wherein:
during an operation, at least a part of the suture (A) extends through the sliding loop (A2), and the sliding loop (A2) is pulled to be shrunk and abuts with the outer circumference of the shaft (22);
the sliding loop (A2) continues to be shrunk and pushes the shaft (22) to move into the through hole (21) to decrease the length of the tying portion (221); and
when the shaft (22) is completely moved inside the through hole (21), and the pre-knot (A1) is detached from the shaft (22) and is tied with the sliding loop (A2) and at least a part of the suture (A) to form a secure knot.

6. The capsule knotting module as claimed in claim 4, wherein a tying portion (221) is defined on at least a part of the shaft (22) protruding from the through hole (21), and a length of the tying portion (221) is varied according to a relative position of the shaft (22) and the through hole (21), wherein:
during an operation, at least a part of the suture (A) extends through the sliding loop (A2), and the sliding loop (A2) is pulled to be shrunk and abuts with the outer circumference of the shaft (22);
the sliding loop (A2) continues to be shrunk and pushes the shaft (22) to move into the through hole (21) to decrease the length of the tying portion (221); and
when the shaft (22) is completely moved inside the through hole (21), and the pre-knot (A1) is detached from the shaft (22) and is tied with the sliding loop (A2) and at least a part of the suture (A) to form a secure knot.

7. The capsule knotting module as claimed in any one of claims 1 to 3, wherein at least a part of the shaft (22) is securely held in the through hole (21), wherein:
during an operation, at least a part of the suture (A) extends through the sliding loop (A2), and the sliding loop (A2) is pulled to be shrunk and abuts with the outer circumference of the shaft (22);
the operating member (10) is continuously pulled to make the pre-knot (A1) to move toward the front end of the capsule knotting module until the pre-knot (A1) is detached from the shaft (22); and
the pre-knot (A1) is detached from the shaft (22) and is tied with the sliding loop (A2) and at least a part of the suture (A), and a secure knot is formed.

8. The capsule knotting module as claimed in claim 4, wherein at least a part of the shaft (22) is securely disposed within the through hole (21),
wherein at least a part of the shaft (22) is securely held in the through hole (21),
wherein:
during an operation, at least a part of the suture (A) extends through the sliding loop (A2), and the sliding loop (A2) is pulled to be shrunk and abuts with the outer circumference of the shaft (22);
the operating member (10) is continuously pulled to make the pre-knot (A1) to move toward the front end of the capsule knotting module until the pre-knot (A1) is detached from the shaft (22); and
the pre-knot (A1) is detached from the shaft (22) and is tied with the sliding loop (A2) and at least a part of the suture (A), and a secure knot is formed.

9. The capsule knotting module as claimed in any one of claims 1 to 3, wherein the knotting end of the suture (A) is connected with a surgical needle (B).

10. The capsule knotting module as claimed in claim 4, wherein the knotting end of the suture (A) is connected with a surgical needle (B).
